(19) Europäisches Patentamt / European Patent Office / Office européen des brevets

(11) **EP 4 466 061 B1**

(12) **EUROPEAN PATENT SPECIFICATION**

(45) Date of publication and mention
of the grant of the patent:
**22.10.2025 Bulletin 2025/43**

(21) Application number: **23701614.2**

(22) Date of filing: **18.01.2023**

(51) International Patent Classification (IPC):
*A61N 1/36* (2006.01)   *A61B 5/37* (2021.01)
*A61B 5/383* (2021.01)   *A61B 5/374* (2021.01)
*A61B 5/00* (2006.01)   *A61B 5/293* (2021.01)
*A61N 1/05* (2006.01)

(52) Cooperative Patent Classification (CPC):
**A61N 1/36139; A61B 5/293; A61B 5/37;**
**A61B 5/374; A61B 5/4064; A61B 5/4082;**
**A61B 5/4094; A61B 5/4848; A61B 5/7225;**
**A61B 5/7257; A61B 5/7264;** A61N 1/0534;
A61N 1/36062; A61N 1/36064; A61N 1/36067;
(Cont.)

(86) International application number:
**PCT/IB2023/050434**

(87) International publication number:
**WO 2023/139503 (27.07.2023 Gazette 2023/30)**

(54) **DEVICE FOR ADAPTIVE TREATMENT OF NEUROLOGICAL DISEASES**

VORRICHTUNG ZUR ADAPTIVEN BEHANDLUNG NEUROLOGISCHER ERKRANKUNGEN

DISPOSITIF DE TRAITEMENT ADAPTATIF DE MALADIES NEUROLOGIQUES

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **20.01.2022 IT 202200000965**

(43) Date of publication of application:
**27.11.2024 Bulletin 2024/48**

(73) Proprietor: **Newronika S.p.A.**
**20121 Milano (IT)**

(72) Inventors:
• **ROSSI, Lorenzo**
  **20131 Milano (IT)**
• **ARLOTTI, Mattia**
  **47924 Rimini (IT)**

(74) Representative: **Mati, Silvia et al**
**Thinx S.r.l.**
**P.le Luigi Cadorna, 10**
**20123 Milano (IT)**

(56) References cited:
US-A1- 2012 016 435   US-A1- 2018 078 770
US-A1- 2018 280 699   US-A1- 2020 108 253
US-A1- 2020 188 675

EP 4 466 061 B1

(52) Cooperative Patent Classification (CPC): (Cont.)
A61N 1/36082

**Description**

**TECHNICAL FIELD**

The present disclosure generally relates to the field of treatment of neurological disease, and in particular to devices for treating neurological diseases based on adaptive stimulation.

The present invention is defined by the appended claims.

**BACKGROUND**

Invasive electrical stimulation is nowadays a therapeutic option for a variety of neurological diseases, including movement disorders, psychiatric disorders, and pain. Examples of invasive brain stimulation devices are deep brain stimulation (DBS) systems which are mainly used in the treatment of Parkinson's Disease, Dystonia, Essential Tremor, Epilepsy and Obsessive-Compulsive Disorders, and spinal cord stimulators (SCS) used for pain management. Deep brain stimulation (DBS) systems are used in various industries including medical diagnostics or medical treatments, due to number of advantages.

For example, deep brain stimulation can deliver electrical stimulation to neural structures of the central nervous system of a patient to modulate neural activity. Conventional deep brain stimulation is often programmed by a physician for a predefined stimulation setting which remains constant over time. By considering that the stimulation usually consists of a train of electric pulses of square form with an amplitude, a pulse width and a pulse frequency, the stimulation setting sets the value of at least one of the above-mentioned parameters (stimulation amplitude, stimulation pulse width and stimulation frequency). Neurological diseases, however, are progressive and often the symptomatology fluctuates over time. Accordingly, dealing with neurological disorders, manage the symptoms and correctly titrate the therapy is not a simple task because it requires to assess the clinical state of the patient objectively and recurrently. For instance, the pain sensation of a patient cannot be measured quantitatively but only through qualitative scales and assessments. Moreover, pain sensation may vary over time.

Such patients benefit from an adaptive and patient-specific stimulation which compensates for symptoms fluctuations by adjusting the stimulation settings in real time based on neuro-physiological control variables. However, the effectiveness of adaptive techniques is strictly related to the stability of the neuro-physiological signal which can be influenced by the electrode-tissue interface degradation and relative position and the underlying evolving pathophysiological mechanism. In conclusion, while adaptive techniques are suitable for managing symptom fluctuations, they do not deal with disease progression and signal degradation over time.

Due to this limitation, in known devices for adaptive deep brain stimulation, maintaining robustness and effectiveness of the adaptive control over time remains a quest. Analogous considerations apply to spinal cord stimulators. Thus, there is a need for new and improved devices for adaptive treatment of neurological diseases.

Documents US 2018/078770, US 2020/108253 and US 2012/016435 describe brain stimulation systems which implement a cascade architecture in which a second control logic tunes the stimulation parameters calculated by a first control logic.

**SUMMARY OF THE INVENTION**

Applicant contemplated the problem of overcoming the above-mentioned issues, and, in particular, of eliminating the need of an initialization and a long-term tuning of a device for adaptive treatment of neurological diseases to set and maintain over time a patient-tailored therapy delivering optimized stimulation.

Within the scope of the above problem, the Applicant considered the objective of devising a device capable of stimulating the neural tissue according to a control logic, collecting the neural signals generated in response to stimulation, and adjusting the stimulation parameters and tuning the control logic based on the collected neural signal.

Accordingly, present invention relates to a device for adaptive treatment of neurological diseases as defined by claim 1, said device comprising an implantable electrode configured to sense neural activity signals and apply electrical stimulation signals, and a processing and stimulation unit connected to the implantable electrode, wherein the processing and stimulation unit at least comprises:

a stimulation module configured to generate a stimulation signal to be carried at the implantable electrode, the stimulation signal being characterized by at least one stimulation parameter;

an acquisition module configured to record neural activity signal records of neural activity signals sensed by the implantable electrode; and

a processing module configured to process the neural activity signal records recorded by the acquisition module based on a first control logic and to tune the at least one stimulation parameter based on at least one neural activity signal record processed according to the first control logic, the first control logic being a function depending on at least one signal feature of the neural activity signal records, being based on at least one control parameter and being made of a plurality of different function pieces, wherein each function piece of the plurality of different function pieces is related to a respective range of the at least one signal feature, wherein the processing module is configured to process the neural activity signal record recorded by the acquisition module based on a second control logic and to tune the at least one control parameter of the first control logic based on the at least one neural activity signal record processed according to the second control logic.

Within the present description and in the appended claims with the expression "function made of a plurality of different function pieces" it is intended to refer to a piecewise function, namely a function defined by multiple sub-functions, wherein each sub-function is a different function piece which applies to a different interval in the domain.

The Applicant found that by iteratively adjusting the control parameters characterizing the first control logic based on the acquired neural activity signals makes it possible both, to self-initialize and self-tune the device for deep brain stimulation when starting the therapy, and to have a robust and long-lasting optimized stimulation.

The Applicant observed that while fluctuations in the symptomatology of the patient are a real-time occurrence (happening in the range of a day or less), disease progression and changes at the electrode-tissue interface occur within a much longer time frame (some days, weeks or months). Therefore, the tuning of the control parameters of the first control logic need to be based on slower variation trends experienced by the neural activity signal, in contrast to the real-time variations which are used to adjust the stimulation parameters.

The Applicant also noted that a control logic aiming to drive stimulation parameters in real-time based on static control parameters cannot adequately fulfill the requirement of robustness in the long term. Accordingly, Applicant realized that tunable control parameters are necessary to take account of long-term signal changes related to electrode-tissue interface modifications and/or disease progression.

All the above particularly applies in case of control logics based on piecewise functions (functions made of a plurality of different function pieces), namely, as known in the art, functions defined by multiple sub-functions, wherein each sub-function applies to a different interval in the domain. As known in the art, the different domain intervals of piecewise control logics applied in adaptive treatment of neurological diseases are selected based on and strongly depend on the patient's conditions in the absence and in the presence of medication and/or stimulation.

The Applicant observed that such conditions are not constant over time, but, on the contrary, may change over longer time periods (days, weeks, months) thereby making the selected piecewise control logic not more optimal for the real-time setting of the stimulation parameters.

An aspect of the present disclosure relates to an exemplary method for controlling a device for adaptive treatment of neurological diseases comprising the steps of: based on a first control logic, processing neural activity signal records recorded by an acquisition module of a device for adaptive treatment of neurological diseases, wherein the first control logic is a function depending on at least one signal feature of the neural activity signal records, is based on at least one control parameter and is made of a plurality of different function pieces, each function piece of the plurality of different function pieces being related to a respective range of the at least one signal feature,

tuning at least one stimulation parameter of a stimulation signal based on at least one neural activity signal record processed according to the first control logic;
based on a second control logic, processing neural activity signal records recorded by the acquisition module, and tuning the at least one control parameter of the first control logic based on at least one neural activity signal record processed according to the second control logic.

Advantageously, the method for controlling a device for adaptive treatment of neurological diseases achieves the same advantages as described with reference to the device for adaptive treatment of neurological diseases according to the invention.

The present invention is defined by the appended claims.

Embodiments, examples, methods and aspects described hereinafter are only of exemplary nature and presented for better understanding the invention.

Generally, in some variations, the processing and stimulation unit may further be configured to define a stimulation parameter window comprised between a minimum of at least a stimulation parameter Amin (amplitude, pulse width, frequency or a combination thereof) eliciting a detectable clinical benefit to the patient and a maximum stimulation parameter Amax (amplitude, pulse width, frequency or a combination thereof) before eliciting a side effect to the patient. In some embodiments, the stimulation parameter window (Amax, Amin) may be entered as non-tunable parameter.

Advantageously, setting a stimulation parameter window instead of a specific stimulation parameter value facilitates and accelerates the initialization phase performed by the physicians.

In some variants, the at least one stimulation parameter of a stimulation signal is tuned based on a timeseries of collected neural activity signal records which is shorter than a timeseries of collected neural activity signal records based on which the at least one control parameter of the first control logic is tuned.

In this way it becomes possible to compensate for disease progression and changes at the electrode-tissue interface which occur within a much longer time frame (some days, weeks or months) compared to fluctuations in the symptomatology of the patient, happening in the range of less than a day. Accordingly, tuning the control parameters of the first control logic according to the second control logic which takes account of a longer timeseries of collected neural activity signal records allows to take account of long-term signal changes related to electrode-tissue interface modifications and/or disease progression.

In some variants, at least one function piece of the plurality of function pieces of the first control logic may be a function depending on the at least one signal feature $F_i$ ($i = 1, ...., N$) of the neural activity signal records. The at least one function piece of the plurality of function pieces may be a linear function of the at least one signal feature $F_i$ of the neural activity signal records.

The first control logic may comprise a first piece of function in which the stimulation parameter A is proportional to the signal feature $F$, with respect to a of a first timeseries of collected neural activity signal features $F_1$. The first piece of function may be related to a first range of neural activity signal features of the first collected timeseries ($C_2 < F_1 < C_1$). The first control logic may comprise a second piece of function defining an upper limit Amax of the stimulation parameter A. The second piece of function may be related to a second range of the signal feature ($F_1 > C_1$). The first control logic may further comprise a third piece of function defining a lower limit Amin of the stimulation parameter A. The third piece of function may be related to a third range of the signal feature ($F_1 < C_2$). The upper and lower limits of the stimulation parameter A may define the stimulation parameter window (Amax, Amin).

The first control logic may be as follows:

$$
A\,(F) = \begin{cases} A_{max} & for\ F_1 > C_1(F_2) \\ \dfrac{F_1 - C_2(F_2)}{C_1(F_2) - C_2\,(F_2)} * (A_{max} - A_{min}) + A_{min} & for\ C_2(F_2) < F_1 < C_1(F_2) \\ A_{min} & for\ F_1 < C_2(F_2) \end{cases}
$$

In this case the first control logic is characterized by a first control parameter $C_1$ and a second control parameter $C_2$ which define the first range for a first timeseries of collected neural activity signal features $F_1$ and which may be adjusted based on the second control logic, and particularly based on a second timeseries of collected neural activity signal features $F_2$.

In some implementations, the processing and stimulation unit of the implantable device may be further configured to extract spectral features within a frequency band of the neural activity signal records that are recorded during a predefined time period. The frequency band may be a low-frequency band, the alpha frequency band, or the beta frequency band and gamma frequencies. The frequency band may be determined by identifying a peak in the extracted spectral features and defining the frequency band as a frequency range around the peak, preferably centered at the peak. In some variants, the frequency band may be preset (hard coded) or entered as non-tunable parameter.

In some implementations, the at least one signal feature of the neural activity signal records may be a spectral feature of the neural activity signal records within the frequency band, preferably a spectral power of the neural activity signal records within the frequency band.

Accordingly, the first control logic may comprise a first piece of function in which the stimulation parameter A is proportional to the power P of the signal in the frequency band. The first piece of function may be related to a first power range ($Pmin < P < Pmax$). The first control logic may comprise a second piece of function defining an upper limit Amax of the stimulation parameter A. The second piece of function may be related to a second power range ($P > Pmax$). The first control logic may further comprise a third piece of function defining a lower limit Amin of the stimulation parameter A. The third piece of function may be related to a third power range ($P < Pmin$).

Thus, the first control logic may be as follows:

$$
A\,(P) = \begin{cases} A_{max} & for\ P > Pmax(P_j) \\ \dfrac{P - Pmin(P_j)}{Pmax(P_j) - Pmin(P_j)} * (A_{max} - A_{min}) + A_{min} & for\ Pmin(P_j) < P < Pmax(P_j) \\ A_{min} & for\ P < Pmin(P_j) \end{cases}
$$

In this case the first control logic is characterized by a first control parameter which corresponds to a maximum spectral power Pmax and a second control parameter which corresponds to a minimum spectral power. The first and second control parameters may be adjusted based on the second control logic, and particularly based on a different timeseries of power P of the signal in the frequency band than the timeseries based on which the stimulation parameter A is adjusted.

In some embodiments, the first control parameter Pmax may be initialized by setting it equal to a power in the frequency band of the spectrum of the neural activity signal measured preferably in the absence of medication and the second control parameter Pmin may be initialized by setting it equal to a power in the frequency band of a background activity spectrum fitted to the spectrum of the neural activity signal in any medication or stimulation condition.

The background activity spectrum may be estimated by fitting the spectrum of the neural activity signal as the following noise function: $n(f) = \frac{k_1}{f^{\alpha}}$ , namely as white noise ($\alpha = 0$) or pink noise ($\alpha = 1$) or red noise ($\alpha = 2$), or by calculating the fractal components of the neural activity signal (thereby separating the not-oscillatory part from the oscillatory part).

In some variants, the second control logic may be implemented as Bollinger bands computing, with the first control parameter Pmax being set equal to an upper band limit and the second control parameter Pmin being set equal to a lower band limit. Both the upper and the lower band limits may be computed based on power timeseries collected over minutes, hours, days, weeks or months. The computing of Bollinger Bands may be based on a simple moving average of any time periods, such as 30 minutes, an hour, a day, a week, etc. Bollinger bands may alternatively be calculated based on exponential moving average. The upper and lower limits of the Bollinger Band may be a number k of standard deviations (positively and negatively) away from the moving average.

In a preferred embodiment, an average power $\overline{P}$ and an average standard deviation $\overline{\sigma}$ of the frequency band may be calculated with sliding time periods.

In a preferred embodiment, the Bollinger Band upper and lower limits may be computed as:

$$Pmax = \overline{P} + k * \overline{\sigma}$$

$$Pmin = \overline{P} - k * \overline{\sigma}$$

with k={1,...,5}.

Advantageously, Bollinger Bands computing provides for a time-relative setting of the first and second control parameters Pmax and Pmin, thereby allowing a self-tuning of the first control logic.

In some embodiments, the second control logic may be an unsupervised learning model establishing clusters of the neural activity signal records based on its at least one signal feature. The unsupervised learning model may be implemented as a K-means clustering method. The K-means clustering method may assign the signal records to K-clusters based on a distance of the signal feature from a centroid of each cluster, where K is a hyperparameter corresponding to a total number of clusters.

In some embodiments, the at least one signal feature of the neural activity signal records may be the spectral power in the frequency band calculated for each record of neural activity signal and the number of clusters may be two with the control parameters Pmax and Pmin which correspond to a centroid of a respective cluster.

In some variants, the unsupervised learning model may be implemented as exclusive (e.g. K-means), overlapping (e.g. fuzzy K-means), hierarchical or probabilistic (e.g. Gaussian Mixture Models) clustering models. The number of clusters may be pre-defined (e.g. two clusters) or set during and/or after acquisition of the neural activity signal records.

In some embodiments, the second control logic may be a time-based fuzzy controller estimating a new set of control parameters of the first control logic based on the at least one signal feature of the neural activity signal records and a related time slot of the day during which the neural activity signal records have been acquired. The time-based fuzzy controller may group and process the at least one signal feature of the recorded neural activity signals based on the time slot when the respective neural activity signal was recorded.

In some embodiments, the at least one signal feature of the neural activity signal records may be the spectral power in the frequency band calculated for each record of neural activity signal and the control parameters Pmax and Pmin may be equal to the mean value of the spectral power values of a first and a second group of spectral power values, wherein each group of spectral power values comprises the spectral power values relating to neural activity signals recorded during respective time slots of the day.

## BRIEF DESCRIPTION OF THE DRAWINGS

FIG. 1 is a schematic view of an exemplary device for adaptive treatment of neurological diseases according to a preferred embodiment of the present invention;

FIG. 2 is a block diagram of the closed-loop control logic implemented by a device for adaptive treatment of neurological diseases according to a preferred embodiment of the present invention;

FIGS. 3a to 3c are block diagrams of exemplary embodiments of the second control logic implemented by a device for adaptive deep brain stimulation according to the present invention;

FIGS. 4a and 4b are flow charts of the exemplary method for controlling a device for adaptive treatment of neurological diseases according to a first and a second embodiment of the present disclosure, respectively;

FIG. 5 is a flow chart of an initialization routine of the method for controlling a device for adaptive treatment of neurological diseases according to the present disclosure;

FIG. 6 is a graph of the spectrum of the neural signal and the background activity; and

FIG. 7 is a schematic depiction of an exemplary device for adaptive treatment of neurological diseases and a related patient personal controller device.

## DETAILED DESCRIPTION

In the figures and in the following description, identical reference numerals or symbols are used to indicate constructive elements with the same function. Moreover, for the sake of clarity of illustration, it is possible that some reference numerals are not repeated in all of the figures. While examples and variations of the invention are depicted and described herein, it should be understood that there is no intention to limit the invention to the specific examples and variations embodiments described below, but on the contrary, the invention is meant to cover all the modifications or alternative and equivalent implementations which fall within the scope of protection of the invention as defined in the claims.

Expressions like "example given", "etc.", "or" indicate non-exclusive alternatives without limitation, unless expressly differently indicated. Expressions like "comprising" and "including" have the meaning of "comprising or including, but not limited to" unless expressly differently indicated. Further, a "module" as referenced throughout may refer to an assembly of electrical circuitry and/or electrical components that are arranged and connected to perform one or more functions as described herein, and/or may refer to a special purpose computer that is programmed to perform the functions described herein.

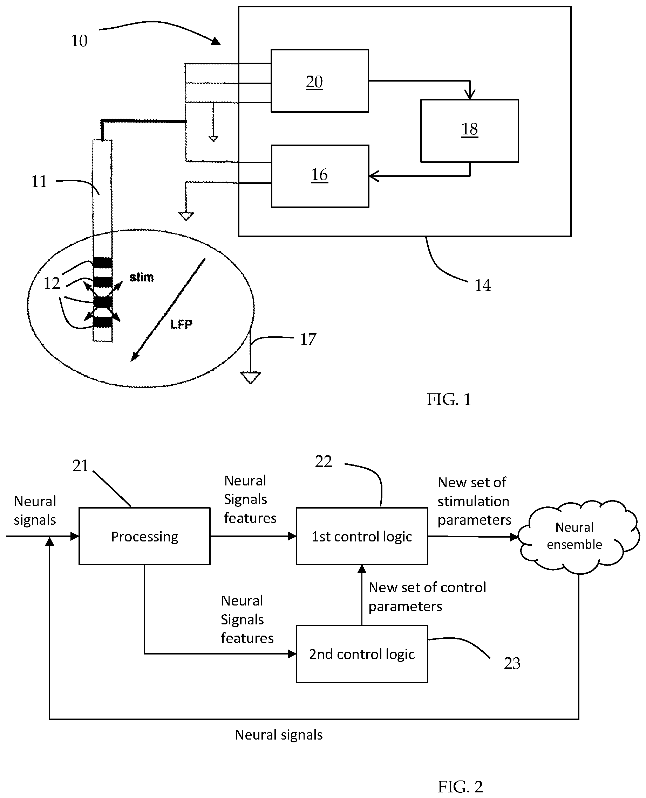

With reference to FIG. 1, one variation of a device for adaptive treatment of neurological diseases is shown, wholly indicated with 10.

In particular, the device illustrated in FIG. 1 is suitable for the adaptive deep brain stimulation being configured to detect biopotentials (e.g., local field potentials or LFPs) from a stimulating electrode or from contiguous electrodes, for correlating such signals to the stimulation effects and/or for adapting stimulation parameters in order to facilitate patient therapy.

The device for adaptive treatment of neurological diseases 10 comprises at least one probe or electro-catheter 11 configured to be implanted in the brain of a patient to administer electrical stimulation. The probe or electro-catheter 11 may comprise at least three metallic contacts or leads accessible through external connections, also called electrodes 12. However, in other variations, the electrodes may not be located on the same electro-catheter (e.g., a device for adaptive DBS may comprise two or more electro-catheters and the electrodes may be located on two different electro-catheters).

The device for adaptive treatment of neurological diseases 10 may comprise one or more implantable probes where each probe may comprise one or more electrodes. The device 10 may also comprise a connector or probe extension for each of the implantable probes. A probe (e.g., probe 11) may have a distal portion and a proximal portion. The one or more electrodes (for delivering electrical stimulation and/or for neural activity data acquisition) are located on the distal portion and one or more connector contacts are located on the proximal portion, and one or more wires within the probe electrically connect the electrodes with the connector contacts. A probe 11 may comprise any number of electrodes 12, for example, 1, 2, 3, 4, 5, 6, 8, 10, 12, 16, 24, 36, 48, 64, 96, etc. and a corresponding number of connector contacts. A probe extension may have a distal portion having a connector block with a receptacle housing enclosing one or more conductive contacts, a proximal portion having stimulation device (e.g., device 10) connector contacts, where each of the stimulation device connector contacts corresponds with a conductive contact in the receptacle housing via one or more wires, and an elongated body between the proximal portion and the distal portion. A probe extension may comprise any number of conductive contacts, for example, 1, 2, 3, 4, 5, 6, 8, 10, 12, 16, 24, 36, 48, 64, 96, etc. and a corresponding number of stimulation device connector contacts. The number of conductive contacts of the probe extension may be the same as, or greater than, the number of electrodes on the probe to which the probe extension is connected. The distal portion of the probe may be implantable into the target brain region, while the proximal portion of the probe may extend outside of the brain tissue and connect with a distal portion of a probe extension. The receptacle housing of the probe extension may be configured to retain the proximal portion of the probe such that the connector contacts of the probe electrically connect with the conductive contacts of the probe extension such that the electrodes at the distal portion of the probe are electrically coupled to the stimulation device connector contacts at the proximal portion of the probe extension. The stimulation device connector contacts may be configured to be coupled to a port or connector of a processing and stimulation unit 14 (e.g., a header interface). In some variations, the receptacle housing may comprise an attachment mechanism to engage or retain the proximal portion of the probe within the receptacle housing. Optionally, the probe extension may comprise a connector sleeve or boot comprising an electrically insulating material that is disposed over at least a portion of the receptacle housing to help electrically isolate the connector contacts of the probe and the conductive contacts of the probe extension from surrounding tissue. The elongated body of the probe extension may have a constant diameter between the distal portion and the proximal portion, or may have a varying diameter along its length. For example, the diameter of a segment of the elongated body may be larger (e.g., thicker) where that segment is intended to be located at the interface between brain tissue and the skull or skin. This may help reduce excessive twisting, torquing, and/or bending of the wires within the elongated body of the probe extension, thereby reducing the mechanical wear on the wires and/or helping to prolong the usable life of the probe extension.

While the device for adaptive treatment of neurological diseases 10 depicted in FIG. 1 comprises a probe 11 having four metallic contacts or electrodes 12, other variations of probes may comprise any number of electrodes (e.g., 1, 2, 3, 4, 5, 6, 7, 8, 9, 10, 12, 16, 18, 20, 25, 30, 36, 48, or more). As described previously, a device for adaptive treatment of neurological diseases 10 may comprise any number of probes (e.g., two or more), where each probe may have any number of electrodes. For example, a device for adaptive treatment of neurological diseases 10 may comprise a first probe with a first electrode and a second probe with a second electrode. In use, the first probe may be implanted in a first brain region and the second probe may be implanted in a second brain region (e.g., for bilateral stimulation). In another variation, a device for adaptive treatment of neurological diseases 10 may comprise two probes, where each probe may have four electrodes (for a total of eight channels) or may have eight electrodes (for a total of sixteen channels).

In one variation, a probe 11 may comprise multiple electrodes where a first electrode is a stimulating electrode that delivers electrical stimulation and a second electrode is a measurement electrode that acquires neural activity signals. For example, a first plurality of electrodes (which may or may not be adjacent to each other) may be used for stimulating and a second plurality of electrodes (which may or may not be adjacent to each other or may be arranged in alternating fashion with the first plurality of electrodes) may be used for acquiring neural activity signals. Alternatively or additionally, the same electrode(s) may be used for both neural activity signal acquisition and electrical stimulation simultaneously or sequentially. DBS probes may comprise one or more cylindrical or disc-shaped electrodes having a height from about 0.5 mm to about 3 mm, e.g., about 1.5 mm, and a diameter from about 0.5 mm to about 2 mm, e.g., about 1.27 mm. In some variations, DBS probes may comprise two or more cylindrical electrodes (for example, 2, 4, 6, 10, 12, 15, 16, 20, etc. or more electrodes). Alternatively or additionally, DBS probes may comprise planar electrodes and/or sharp electrodes having a geometry selected at least in part based on the target neural structure or brain region. The spacing between two electrodes may be from about 0.25 mm to about 2 mm, e.g., about 0.5 mm, and optionally, an insulator may be disposed between two electrodes and/or around an electrode to reduce electrical coupling or crosstalk between electrodes. An insulator may comprise, for example, polyurethane and/or polyimide and/or the like. The electrodes may be made of any metal or any metallic alloy, for example, a platinum-iridium alloy.

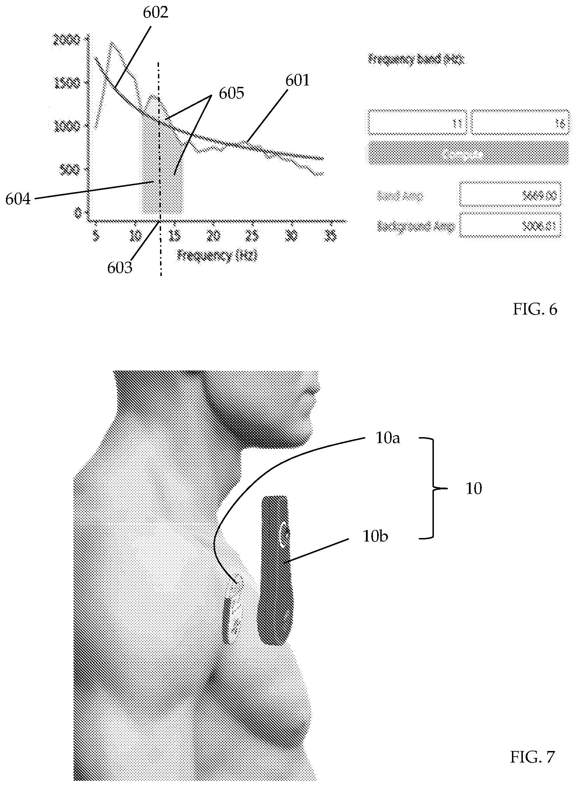

In the embodiment illustrated in FIG. 1, the electrodes 12 are connected to a processing and stimulation unit 14 that comprises three functional modules connected together in a feedback and interoperating configuration: a stimulation module 16, a data acquisition module 20 and a processing module 18. The processing and stimulation unit 14 may be fully comprised in an implantable portion 10a of the device for adaptive treatment of neurological diseases 10 or be distributed between the implantable portion 10a and an external portion 10b. In this second case, raw and/or processed neural activity signal records are wirelessly transmitted from the implantable 10a to the external 10b portion and vice versa. The external portion 10b may be implemented as patient personal controller device. The implantable 10a and external 10b portions of the device for adaptive treatment of neurological diseases 10 are described in detail below and schematically depicted in FIG. 7.

In one variation, the processing and stimulation unit 14 may comprise sixteen channels, which may be connected to two probes each having eight electrodes, or four probes each having four electrodes, or eight probes each having two electrodes, etc. There may be fewer electrodes than channels, for example, although the processing and stimulation unit 14 may be configured to accommodate sixteen channels (e.g., for sixteen stimulation and/or LFP acquisition electrodes), a particular instance of a device for adaptive treatment of neurological diseases 10 or DBS system may comprise eight electrodes (e.g., two probes each having four electrodes) or four electrodes (e.g., a single probe having four electrodes).

The stimulation module 16 is adopted to generate a stimulation signal and to send it to the electrodes 12. The stimulation module 16 may comprise pulse or function generator comprising a voltage source and/or current source and circuitry configured to produce electrical pulses with certain parameter values determined by a user and/or controller, and may also comprise wires that transmit the electrical pulses to the probe which deliver the electrical pulses to the brain region.

In some variations, the stimulation module may comprise a waveform generator (e.g., a pulse or function generator), a current controller, and a multiplexer, one or more of which may be configured to receive command signals from the processing module 18. The command signals may comprise electrical stimulation parameter data, including, but not limited to, stimulation amplitude, pulse width, pulse frequency, duty cycle, and/or the specific probe(s) and/or electrode(s) from which electrical stimulation with the specified parameters is to be delivered. The current controller may be configured to set an electrical stimulation amplitude specified by the command signals, and/or the waveform generator may be configured to generate current or voltage pulses having the pulse width and/or pulse frequency specified by the command signals. The multiplexer may be configured to electrically connect the probes and/or electrodes specified by the command signals with the current controller and/or waveform generator. In some variations, the multiplexer may comprise a multiplexer array that may be configured according to command signals from the main processor so that the electrical pulses from the waveform generator may be directed to the selected probes and/or electrodes. The connectivity between the waveform generator and the electrodes may be arranged by the multiplexer in a monopolar stimulation configuration and/or a bipolar stimulation configuration. In a monopolar configuration, one or more electrodes may be connected to one or more active (e.g., positive) terminals of the waveform generator (with a return pad placed elsewhere on a patient). In a bipolar configuration, a first set of one or more electrodes may be connected to one or more active (e.g., positive) terminals of the waveform generator while a second set of one or more electrodes (e.g. distinct from the first set of electrodes) may be connected to one or more return (e.g., negative) terminals of the waveform generator.

In some variations, the stimulation module 16 may be configured to generate a stimulation signal that may be characterised by a set of parameters, and to transmit the stimulation signal to one or more of the electrodes 12. For example, the stimulation module 16 may comprise a pulse generator having a current source (and/or voltage source) that generates electrical signals that have parameters specified by a user and/or the processing module. In some variations, a pulse generator may form output pulses having specified amplitude, frequency and/or pulse width or duration values. Optionally, a pulse generator may generate a pulse sequence having two pulses or more pulses repeated with a duty cycle specified by a user and/or the processing module 18, and the processing module 18 may adjust the pulse duty cycle in accordance with one or more properties of the acquired neural activity signals (e.g., any of the patterns or properties described herein).

The data acquisition module 20 is responsible for the acquisition of a signal representative of the cerebral activity coming from the brain of the patient, e.g. LFP signals that may represent the cerebral activity in the brain region where the probe 11 is implanted. The acquisition module 20 is in electrical communication with the probe 11 which may be, in some variations, the same probe used to electrically stimulate the brain region. The acquisition module 20 and/or the probe 11 may be configured to acquire neural activity signals, such as local-field potentials (LFPs), resulting from the activity of the brain region in proximity to the probes 11. The acquisition module 20 may comprise an acquisition processor and memory that stores and analyzes the acquired neural activity signals.

The processing module 18 implements an adaptive control of the stimulation module 16 based on the signal acquired by the acquisition module 20. The processing module may have circuitry configured to facilitate communication between the acquisition module 20 and the stimulation module 16, coordinate signaling between the acquisition module 20 and the stimulation module 16, and/or to perform additional computations on the acquired neural activity signals.

The processing module 18 may be part of either the acquisition module or the stimulation module, or may be a separate module. In some variations, the processing module comprises circuitry configured to regulate/coordinate the operation of the stimulation module based on signals from the acquisition module (e.g., based on LFP signals indicative of neural activity). The processing module may have a module (main) processor and memory that analyzes and stores the acquired neural activity signals and/or signals from the acquisition module. In some variations, the processing module may comprise circuitry that regulates the power supplied to the stimulation module, for example, in coordination with the electrical stimulation parameters determined by the acquisition module and/or the acquired neural activity signals. The properties or parameters of the electrical stimulation may be determined by the acquisition module and/or the processing module. For example, the processors of the acquisition module and/or the processing module may analyze the acquired and/or stored neural activity signals to identify variations or changes in the patterns or characteristics of neural activity signals. The processing module may provide command signals to the pulse generator of the stimulation module to change the parameters of the electrical stimulation according to the changes in the neural activity signals detected or extracted by the acquisition module. The processing module may also comprise a battery (e.g., a rechargeable battery), and circuitry configured to charge and/or measure the charge remaining on the battery.

For example, the processing module may comprise a rechargeable battery, an inductive link for charging the battery and an inductive coil for facilitating the energy transfer between an external charging device and the stimulation device (which may be implanted in the patient). Optionally, the processing module may comprise wireless transmission interface (e.g., a transceiver) including an RF chip and an RF antenna for signal transmission between the implantable stimulation device and an external device. In some variations, the acquisition module may comprise a processor that is configured to calculate the spectral power values of acquired neural activity signals, and the calculated power values may be transmitted to the processing module, and the processing module processor may be configured to derive stimulation parameters according to the power values and general command signals to the pulse generator to adapt or adjust the parameters of the electrical stimulation. Optionally, the processing module may comprise additional sub-modules with circuitry configured for power supply management, electrode impedance checking, and/or calibration and/or diagnostic analyses (e.g., troubleshooting) of the stimulation module.

Going back to the acquisition module 20 of FIG. 1, its main function is to measure the electric field variations of the local biopotentials directly sensing the difference between the electric potentials referred to a common electrode 17 and to amplify such difference so as to reach a voltage level useful for the analog-to-digital conversion necessary for the signal processing.

Accordingly, the acquisition module 20 may comprise input ports that are each connected to different electrodes 12 on the probe 11 and electrical circuits that are configured to measure the electric field variations of the local biopotentials or local field potentials (LFPs) based on the signals from the input ports. Electrical circuits of the acquisition module may comprise one or more processing units or processors (e.g., a CPU, and/or one or more field-programmable gate arrays, and/or one or more application-specific integrated circuits) that may be configured to perform computational operations, one or more memory elements, one or more amplifiers, one or more filters, and/or one or more analog-to-digital converters.

FIG. 2 is a functional block diagram showing the operations implemented by the acquisition 20 and processing 18 modules of the processing and stimulation unit 14. As mentioned before, at block 21 the acquisition module 20 may calculate a set of signal features of acquired neural activity signals, e.g., the spectral power values. The spectral power values may be transmitted to the processing module 18, and the processing module processor may apply a first control logic (shown at block 22) to the received spectral power values to derive stimulation parameters to adapt or adjust the parameters of the electrical stimulation.

The first control logic of block 22 may be a function depending on at least one signal feature $F_i$ ($i = 1, \ldots, N$) of the neural activity signal records and may be based on at least one control parameter $C_j$ ($j = 1, \ldots, M$). The first control logic may be made of a plurality of different function pieces, wherein each function piece of the plurality of different function pieces is related to a respective range of the at least one signal feature $F_i$. At least one function piece of the plurality of function pieces may be a function depending on the at least one signal feature $F_i$ of the neural activity signal records. The at least one function piece of the plurality of function pieces may be a linear function of the at least one signal feature $F_i$ of the neural activity signal records. At least one further function piece of the plurality of function pieces may be a constant value.

For instance, in one implementation, the first control logic may comprise a first piece of function in which the stimulation parameter A is proportional to the signal feature F , with respect to a of a first timeseries of collected neural activity signal features $F_1$. The first piece of function may be related to a first range of neural activity signal features of the first collected timeseries ($C_2 < F_1 < C_1$). The first control logic may comprise a second piece of function defining an upper limit Amax of the stimulation parameter A. The second piece of function may be related to a second range of the signal feature ($F_1 > C_1$). The first control logic may further comprise a third piece of function defining a lower limit Amin of the stimulation parameter A. The third piece of function may be related to a third range of the signal feature ($F_1 < C_2$). The upper and lower limits of the stimulation parameter A may define a stimulation parameter window (Amax, Amin).

Accordingly, the first control logic may be as follows:

$$A\left(F\right)=\begin{cases} A_{max} & for\ F_1 > C_1(F_2) \\ \dfrac{F_1 - C_2\left(F_2\right)}{C_1(F_2) - C_2(F_2)} * \left(A_{max} - A_{min}\right) + A_{min} & for\ C_2(F_2) < F_1 < C_1(F_2) \\ A_{min} & for\ F_1 < C_2(F_2) \end{cases}$$

In this case the first control logic is characterized by a first control parameter $C_1$ and a second control parameter $C_2$ which define the first range for a first timeseries of collected neural activity signal features $F_1$ and which may be adjusted based on the second control logic, and particularly based on a second timeseries of collected neural activity signal features $F_2$ processed according to the second control logic.

For instance, in one implementation, the at least one signal feature of the neural activity signal records may be a spectral feature within a frequency band, preferably a power of the neural activity signal in the frequency band. The frequency band may be a low-frequency band, the alpha frequency band, or the beta frequency band and gamma frequencies. The frequency band may be preset (hard coded) or entered as non-tunable parameter.

Accordingly, the first control logic may comprise a first piece of function in which the stimulation parameter A is proportional to the power of the signal in the frequency band. The first piece of function may be related to a first power range (*Pmin < P < Pmax*). The first control logic may comprise a second piece of function defining an upper limit Amax of the stimulation parameter A. The second piece of function may be related to a second power range (*P > Pmax*). The first control logic may further comprise a third piece of function defining a lower limit Amin of the stimulation parameter A. The third piece of function being related to a third power range (P < *Pmin).*

In this implementation, the first control logic may be as follows:

$$A\,(P) = \begin{cases} A_{max} & for\ P > Pmax(P_j) \\ \dfrac{P - Pmin(P_j)}{Pmax(P_j) - Pmin(P_j)} * (A_{max} - A_{min}) + A_{min} & for\ Pmin(P_j) < P < Pmax(P_j) \\ A_{min} & for\ P < Pmin(P_j) \end{cases}$$

In this case the first control logic is characterized by a first control parameter corresponding to a maximum spectral power Pmax and a second control parameter corresponding to a minimum spectral power Pmin.

In some implementations, the at least one a signal feature can be one of, or any combination of, a signal amplitude, a signal phase, an entropy, an inter or intra signal coherence, an inter or intra phase amplitude coupling, a fractal spectrum, a fractal dimension, a phase locking value, a modulation index, a kurtosis, a fluctuation index etc.

The processing module may be further configured to process the neural activity signal records received from the acquisition module based on a second control logic (shown at block 23) and to tune the at least one control parameter Pmin,Pmax of the first control logic of block 22 based on neural activity signal records processed according to the second control logic.

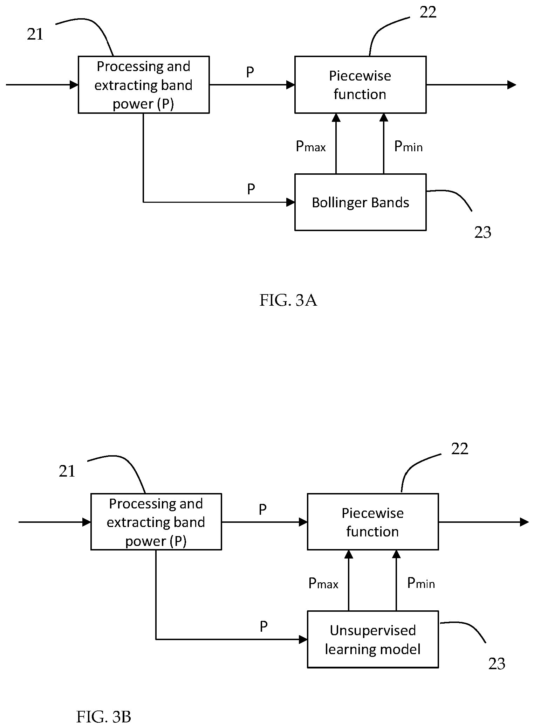

FIGS. 3A to 3C are examples of the second control logic 23 that the processing module 18 may use to tune the control parameters Pmin,Pmax which characterize the first control logic.

In the example of FIG. 3A, the second control logic may be implemented as Bollinger bands computing, with the first control parameter Pmax being set equal to an upper band limit and the second control parameter Pmin being set equal to a lower band limit. Both the upper and the lower band limits may be computed based on power timeseries collected over minutes, hours, days, weeks or months.

Computing of Bollinger Bands may be based on a simple moving average of any time periods, such as 30 minutes, an hour, a day, a week, etc. Bollinger bands may alternatively be calculated based on an exponential moving average. The upper and lower limits of the Bollinger Band may be a number k of standard deviations (positively and negatively) away from the moving average.

An average power $\overline{P}$ and an average standard deviation $\overline{\sigma}$ of the frequency band may be calculated by the second control logic with sliding time periods, e.g., sliding time periods of 24 hours.

Accordingly, after 24 hours at every time step (e.g., every minute) the processing module may compute the Bollinger Band upper and lower limits based on the data collected during the last 24 hours.

The Bollinger Band upper and lower limits may be computed as:

$$Pmax = \overline{P} + k * \overline{\sigma}$$

$$Pmin = \overline{P} - k * \overline{\sigma}$$

with k={1,...,5}.

The time periods for the moving average and standard deviation calculations, and the width of the Bollinger Band given by factor k are hyperparameters of the Bollinger Band computing.

In some variants, the Bollinger Band computing may be applied to the control parameters of the first control logic which define a threshold and are dynamically tunable. The first control logic may control switching ON and OFF the stimulation based on a threshold value of the at least one signal feature of the neural activity signal records. The threshold value may be calculated as the upper or the lower limit of the Bollinger Band.

In the example of FIG. 3B, the second control logic 23 may be implemented as an unsupervised learning model establishing clusters of the neural activity signal records based on its at least one signal feature. The unsupervised learning model may be implemented as a K-means clustering method. The K-means clustering method may assign the signal records to K-clusters based on a distance of the signal feature from a centroid of each cluster, where K is a hyperparameter corresponding to a total number of clusters.

By way of example, the at least one signal feature of the neural activity signal records may be the spectral power in the frequency band calculated for each record of neural activity signal and the number of clusters may be two with the control parameters Pmax and Pmin which correspond to a centroid of a respective cluster. Accordingly, the calculated spectral power which is closer to a cluster centroid will be classified under the corresponding cluster. The value of each centroid is updated after the assignment of the calculated spectral power to a respective cluster. The control parameters Pmax and Pmin determined during an initialization step provide the starting values of the cluster centroids and will iteratively change with the calculated spectral power values added to the respective cluster. This allows tuning of the control parameters Pmax and Pmin over time based on the development of the recorded neural activity signal. Alternatively, the values of the centroid can be initialized randomly.

In some variants, the unsupervised learning model may be implemented as exclusive (e.g., k-means), overlapping (e.g., fuzzy k-means), hierarchical or probabilistic (e.g., Gaussian Mixture Models) clustering models. The number of clusters may be pre-defined (e.g., two clusters) or set during and/or after acquisition of the neural activity signal records.

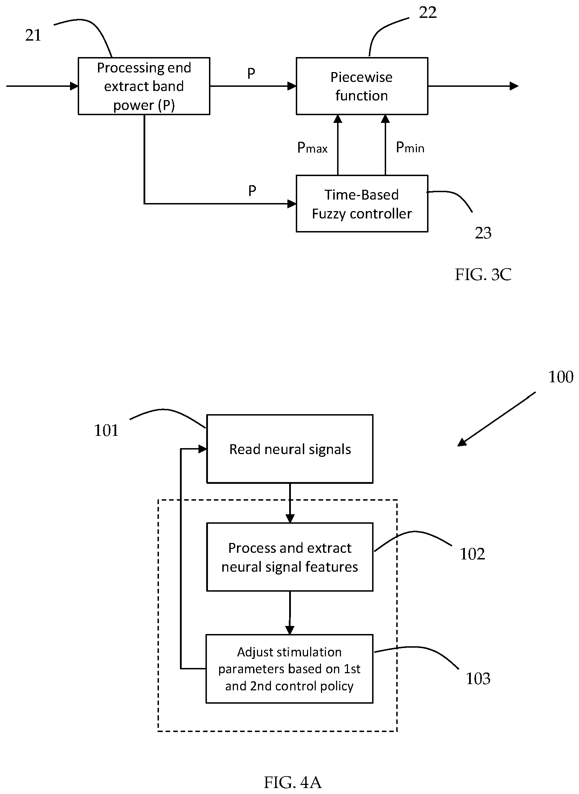

In the example of FIG. 3C, the second control logic 23 may be implemented as a time-based fuzzy controller estimating a new set of control parameters of the first control logic based on the at least one signal feature of the neural activity signal records and a related time slot of the day of its recording. The time-based fuzzy controller may group and process the at least one signal feature of the recorded neural activity signals based on the time slot of the day when the respective neural activity signal was recorded.

For instance, in one implementation the signal features of the neural activity signals recorded between the 10 am - 12 pm and 2 pm - 4 pm may be grouped to belong to a first group, and the signal features of the neural activity signals recorded between 8 am - 10 am and 12 pm - 2 pm may be grouped to belong to a second group. By way of example, the at least one signal feature of the neural activity signal records may be the spectral power in the frequency band calculated for each record of neural activity signal. The control parameters Pmax and Pmin may be equal to the mean value of the spectral power values of the first and the second group, respectively. In a real-time application scenario, the control parameters Pmax and Pmin may be updated each day, or week, or month. The time slots of the day for grouping the signal features of the neural activity signals may be pre-set.

FIG. 4A is a first exemplary method 100 for controlling a device for adaptive treatment of neurological disorders according to the present dislosure. The method 100 comprises, at 101, the step of acquiring and/or storing neural activity signal records such as, for example, local field potentials (LFPs) for a predefined time period (e.g., 1 day, 10 days, etc.). For example, in some instances, the neural activity signal records during a day (e.g., during regular daily activities) can be acquired and recorded separately for left subthalamic nucleus (STN) and right STN in a frequency range between 5 Hz to 35 Hz. The acquisition may be made in a frequency band for the neural activity signal record selected from a low-frequency band, the alpha frequency band, the beta frequency band, and/or gamma frequencies. The frequency band may be determined by identifying a peak in the extracted spectral features and defining the frequency band as a frequency range around the peak, preferably centered at the peak. In some variants, the frequency band may be preset (hard coded) or entered as non-tunable parameter.

The method 100 comprises, at 102, the step of processing the acquired neural activity signal records and extract neural signal features. For example, in some instances, the method may provide for the extraction of spectral features, such as spectral power, within a frequency band of the neural activity signal records that are recorded during a predefined time period

The extracted signal features may be transmitted, at 103, to the processing module 18 to derive stimulation parameters according to the first and second control logic as described before. In detail, while at least one stimulation parameter of a stimulation signal is tuned based on the first control logic and neural activity signal records, the neural activity signal records are processed according to a second control logic to tune the control parameters of the first control logic.

The at least one stimulation parameter of a stimulation signal is tuned based on a timeseries of collected neural activity signal records which is usually shorter than a timeseries of collected neural activity signal records based on which the control parameters of the first control logic are tuned.

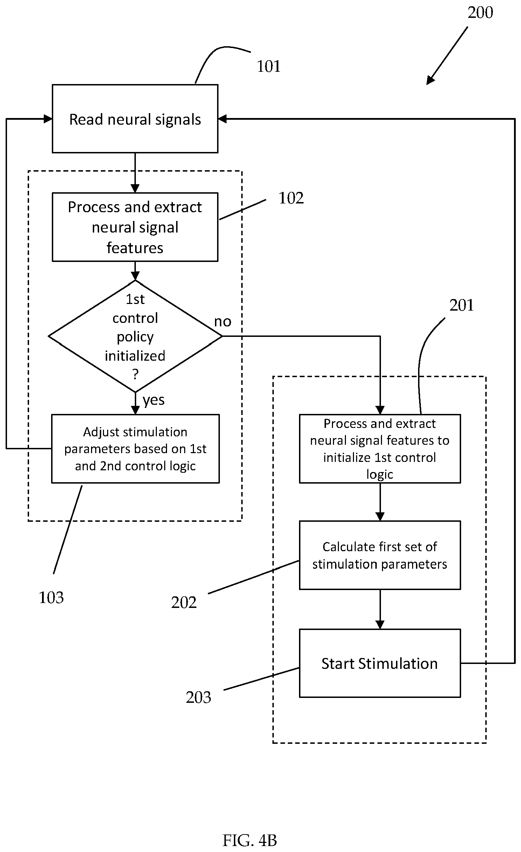

FIG. 4B is a second exemplary method 200 for controlling a device for adaptive treatment of neurological disorders according to the present disclosure. In addition to the steps of the first exemplary method 100, the method 200 comprises a routine for initializing the first control logic based on collected neural signals.

The method 200 comprises, at 101, the step of acquiring and/or storing neural activity signal records and further processing the acquired neural activity signal records and extract neural signal features, at 102. If the process has just started and the first control logic still needs to be initialized, namely its control parameters still have to be set, the step of acquiring and/or storing neural activity signal records and further processing the acquired neural activity signal records are performed preferably in the absence of stimulation and/or medication and, at 201 and 202, the method uses the neural signal features extracted from the neural activity signal records acquired to calculate the control parameters (as will be described in detail below). The adaptive stimulation is finally started at 203.

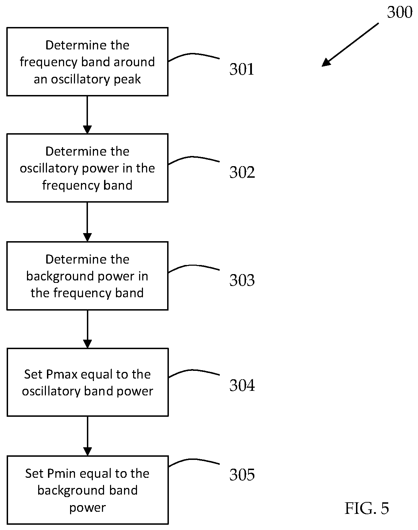

FIG. 5 is an exemplary method 300 for initializing the first control logic, particularly for the case in which the first control logic is a function which depends on the spectral power in a frequency band of the neural activity signal and the control parameters of the first control logic are a first control parameter corresponding to a minimum spectral power Pmin and a second control parameter corresponding to a maximum spectral power Pmax. The method 300 provides for processing neural activity signal records which are acquired in the absence of stimulation. The neural activity signal records are processed at 301 to extract the signal spectrum 601 (shown in FIG.6) and to determine a peak frequency 603 of the neural activity signal records. Based on the position of the peak within the frequency range, a patient-specific frequency band is selected and set to be around the peak and preferably centered around the peak.

At 302, the method 300 determines the spectral power 605 of the neural activity signal records in the selected patient-specific frequency band. Then, the method determines at 303 the spectral power 604 of a background activity spectrum 602 in the same patient-specific frequency band. To this end, a noise function is fitted to the frequency spectrum of the neural activity signal records.

The noise function may be expressed as follow: $n(f) = \frac{k_1}{f^\alpha}$ and may correspond to e.g., white noise ($\alpha = 0$) or pink noise ($\alpha = 1$) or red noise ($\alpha = 2$). In some variants, the background activity spectrum 602 is determined by calculating the fractal components of the neural activity signal (thereby separating the not oscillatory part from the oscillatory part).

Finally, the first and second control parameters Pmax and Pmin are set at 304 and 305, respectively. The first control parameter Pmax is set equal to the spectral power 605 of the neural activity signal records acquired in the absence of stimulation calculated in the selected patient-specific frequency band and the second control parameter Pmin is set equal to the spectral power 604 of the background activity spectrum fitted to the spectrum of the neural activity signal acquired preferably in the absence of stimulation and/or medication calculated in the selected patient-specific frequency band.

FIG. 7 is a schematic depiction of an exemplary method for establishing a wireless connection between an implantable portion 10a of the device for adaptive treatment of neurological diseases 10 (also referred to herein as the 'implantable pulse generator (IPG) device') and an external portion 10b (also referred to herein as the 'patient personal controller device'). The patient personal controller device 10b may transmit power over the wireless connection to charge the IPG 10a. Alternatively, or additionally, the IPG device 10a may transmit/receive neural activity signal records and/or stimulation parameters to/from the patient personal controller device 10b. For example, the IPG 10a may transmit neural activity signal records over the wireless connection to the patient personal controller device 10b, and the personal controller device 10b may transmit stimulation parameters or instructions over the wireless connection to the IPG 10a. In some instances the wireless connection between the implantable device 10a and the patient personal controller device 10b (e.g., a recharger unit of the patient personal controller device) may be established when the patient personal controller device 10b and the implantable device 10a are at a predetermined distance range (e.g., 2 centimeter to 10 centimeter, 1 millimeter to 1 meter, and/or the like) and orientation range. The orientation range can involve, for example, alignment of a vertical orientation of the patient personal controller device to a vertical orientation of the implantable device within 5 rotation degree error margin, 10 rotation degree error margin, and/or the like. The personal controller device 10b may transmit power to the implantable device 10a, and the implantable device 10a may transmit the neural activity signal records to the personal controller device 10b over the first wireless connection.

The foregoing description, for purposes of explanation, specifically refers to DBS applications. However, the disclosed invention may be applied also to different implementations as e.g., SCS for pain treatment. In the case of SCS, the acquisition module may be configured to acquire the neural activity of the spinal nerves in response to electrical stimulation when the electrodes are placed in the spine. The response of the spine to electrical stimulation is the summation of activation of an ensemble of neural fibers, namely evoked compound action potentials (ECAP). The processing module may be configured to calculate a group of signal features of acquired neural activity signals, (e.g., the spectral power of ECAPs). The spectral power values may be transmitted to the processing module, and the processing module processor may apply a first control logic to the received signal feature to derive stimulation parameters to adapt or adjust the parameters of the electrical stimulation. The processing module processor may also apply a second control logic to adjust the control parameter of the first control logic as described above. The aforementioned considerations for the implementation of the first and the second control logic still apply.

The foregoing description, for purposes of explanation, used specific nomenclature to provide a thorough understanding of the invention. However, it will be apparent to one skilled in the art that specific details are not required in order to practice the invention. Thus, the foregoing descriptions of specific variations of the invention are presented for purposes of illustration and description. They are not intended to be exhaustive or to limit the invention to the precise forms disclosed; obviously, many modifications and variations are possible without departing from the scope of the present invention which is defined by the appended claims.

## Claims

1. A device (10) for adaptive treatment of neurological diseases comprising an implantable electrode (11) configured to sense neural activity signals and apply electrical stimulation signals, and
a processing and stimulation unit (14) connected to the implantable electrode (11), wherein the processing and stimulation unit at least comprises:

a stimulation module (16) configured to generate a stimulation signal to be carried at the implantable electrode (11), the stimulation signal being **characterized by** at least one stimulation parameter;

an acquisition module (20) configured to record neural activity signal records of neural activity signals sensed by the implantable electrode; and

a processing module (18) configured to process the neural activity signal records recorded by the acquisition module (20) based on a first control logic (22) and to tune the at least one stimulation parameter (A) based on at least one neural activity signal record processed according to the first control logic (22), the first control logic being a function depending on at least one signal feature (Fi) of the neural activity signal records, being based on at least one control parameter (Cj) and being made of a plurality of different function pieces, wherein each function piece of the plurality of different function pieces is related to a respective range of the at least one signal feature (Fi), wherein the processing module (18) is configured to process the neural activity signal records recorded by the acquisition module (20) based on a second control logic (23) and to tune the at least one control parameter (Cj) of the first control logic (22) based on at least one neural activity signal record processed according to the second control logic (23).

2. The device (10) of claim 1, wherein the processing module (18) is configured to tune the at least one stimulation parameter (A) of a stimulation signal based on a first timeseries of collected neural activity signal records ($F_1$) and and to tune the at least one control parameter (Cj) of the first control logic (22) based on a second timeseries of collected neural activity signal records ($F_2$), with the first timeseries being shorter than the second timeseries.

3. The device (10) of claim 2, wherein the second timeseries of collected neural activity signal records based on which at least one control parameter (Cj) of the first control logic (22) is tuned is a spectral power timeseries collected over minutes or hours or days or weeks or months.

4. The device (10) of any one of claims 1 to 3, wherein the second control logic (23) is a Bollinger bands calculator, with a first control parameter ($C_1$) of the first control logic (22) being set equal to an upper band limit and a second control parameter ($C_2$) of the first control logic (22) being set equal to a lower band limit; or

wherein the second control logic (23) is an unsupervised learning model configured to establish one cluster of the neural activity signal records based on its at least one signal feature (Fi) for each control parameter (Cj) of the first control logic (22), with each control parameter (Cj) corresponding to a centroid of a respective cluster; or
wherein the second control logic (23) is a time-based fuzzy controller configured to estimate at least one control parameter (Cj) of the first control logic (22) based on the at least one signal feature of the neural activity signal records and a related time slot of the day during which the neural activity signal records have been acquired.

5. The device (10) of any one of the preceding claims, wherein at least one function piece of the plurality of function pieces of the first control logic (22) is a function depending on the at least one signal feature (Fi) of the neural activity signal records, preferably a linear function of the at least one signal feature (Fi) of the neural activity signal records.

6. The device (10) of any one of the preceding claims, wherein the first control logic comprises:

- a first piece of function in which the at least one stimulation parameter (A) is proportional to the at least one signal feature (Fi), the first piece of function being related to a first range of the at least one signal feature ($C_2 < F_i < C_1$);
- a second piece of function defining an upper limit (Amax) of the at least one stimulation parameter (A), the second piece of function being related to a second range of the at least one signal feature ($F_i > C_1$); and
- a third piece of function defining a lower limit (Amin) of the at least one stimulation parameter (A), the third piece of function being related to a third range of the at least one signal feature ($F_i < C_2$).

7. The device (10) of claim 6, wherein the first control logic (22) is

$$A\,(F) = \begin{cases} A_{max} & for\ F_1 > C_1(F_2) \\ \dfrac{F_1 - C_2}{C_1 - C_2} * (A_{max} - A_{min}) + A_{min} & for\ C_2 < F_1 < C_1 \\ A_{min} & for\ F_1 < C_2(F_2) \end{cases}$$

wherein the first range of a first timeseries of collected neural activity signal features ($F_1$) of the signal feature comprises an upper ($C_1$) and a lower ($C_2$) range limit which are control parameters of the first control logic (22) tuned based on a second timeseries of collected neural activity signal features ($F_2$) according to the second control logic (23).

8. The device (10) of any one of the preceding claims, wherein the processing and stimulation unit (14) is configured to extract spectral features within a frequency band of the neural activity signal records that are recorded during a predefined time period, the frequency band preferably being a low-frequency band, the alpha frequency band, the beta frequency band or gamma frequencies; and
wherein the at least one signal feature of the neural activity signal records is a spectral feature of the neural activity signal records within the frequency band, preferably a spectral power of the neural activity signal records within the frequency band.

9. The device (10) of claim 8, wherein the first control logic (22) comprises a first piece of function in which the at least one stimulation parameter (A) is proportional to the spectral power (P) of the neural activity signal records within the frequency band, the first piece of function being related to a first power range *(Pmin < P < Pmax)* comprised between a minimum spectral power (Pmin) and a maximum spectral power (Pmax).

10. The device (10) of claim 9, wherein the first control logic (22) comprises a second piece of function defining an upper limit (Amax) of the at least one stimulation parameter (A), wherein the second piece of function is related to a second power range *(P > Pmax)* contiguous to the first power range *(Pmin < P < Pmax)*; and a third piece of function defining a lower limit (Amin) of the at least one stimulation parameter (A), the third piece of function being related to a third power range *(P < Pmin)* contiguous to the first power range *(Pmin < P < Pmax)*.

11. The device (10) of claim 9 or 10, wherein the first control logic (22) is

$$A\,(P) = \begin{cases} A_{max} & for\ P > Pmax(P_j) \\ \dfrac{P - Pmin(P_j)}{Pmax(P_j) - Pmin\,(P_j)} * (A_{max} - A_{min}) + A_{min} & for\ Pmin(P_j) < P < Pmax\,(P_j) \\ A_{min} & for\ P < Pmin(P_j) \end{cases}$$

with Amin and Amax being a lower and an upper limit of the stimulation parameter (A), respectively, and wherein the minimum spectral power (Pmin) and the maximum spectral power (Pmax) are control parameters of the first control logic (22) tuned based on at least one neural activity signal record processed according to the second control logic (23).

12. The device (10) of any one of claims 8 to 11 when dependent on claim 4, wherein the upper band limit and the lower band limit are a number (k) of an average standard deviation ($\overline{\sigma}$) positively and negatively away from an average power ($\overline{P}$), both calculated with sliding time periods, wherein preferably the average power ($\overline{P}$) is calculated as simple moving average of the spectral power of the neural activity signal records within the frequency band or as exponential moving average of the spectral power of the neural activity signal records within the frequency band.

13. The device (10) of any one of claims 4 to 10, wherein the at least one control parameter (Cj) of the first control logic (22) is set equal to the mean value of signal features grouped by the second control logic (23) based on the time slot of the day during which the neural activity signal records have been acquired.

14. The device (10) of any one of the preceding claims, wherein the processing module (18) is further configured to initialize the at least one control parameter (Cj) based on at least one neural activity signal record acquired by the acquisition module (20).

15. The device (10) of any one of the preceding claims, wherein the processing and stimulation unit (14) is configured to define a stimulation parameter window comprised between a lower limit (Amin) of the at least one stimulation parameter (A) and an upper limit (Amax) of the at least one stimulation parameter (A).

**Patentansprüche**

1. Vorrichtung (10) zur adaptiven Behandlung neurologischer Erkrankungen mit einer implantierbaren Elektrode (11), welche dazu eingerichtet ist, neurale Aktivitätssignale zu erfassen und elektrische Stimulationssignale anzulegen, und

eine mit der implantierbaren Elektrode (11) verbundene Verarbeitungs- und Stimulationseinheit (14), wobei die Verarbeitungs- und Stimulationseinheit mindestens umfasst:

ein Stimulationsmodul (16), welches dazu eingerichtet ist, ein Stimulationssignal, welches an der implantierbaren Elektrode (11) zu führen ist, zu erzeugen, wobei das Stimulationssignal durch mindestens einen Stimulationsparameter gekennzeichnet ist;

ein Erfassungsmodul (20), welches dazu eingerichtet ist, Aufzeichnungen von neuronalen Aktivitätssignalen aufzuzeichnen, welche von der implantierbaren Elektrode erfasst werden; und

ein Verarbeitungsmodul (18), welches dazu eingerichtet ist, die von dem Erfassungsmodul (20) aufgezeichneten Aufzeichnungen von neuronalen Aktivitätssignalen basierend auf einer ersten Steuerlogik (22) zu verarbeiten und den mindestens einen Stimulationsparameter (A) basierend auf mindestens einer gemäß der ersten Steuerlogik (22) verarbeiteten Aufzeichnung von neuronalen Aktivitätssignalen einzustellen, wobei die erste Steuerlogik eine Funktion ist, die von mindestens einer Signalcharakteristik (Fi) der Aufzeichnungen von neuronalen Aktivitätssignalen abhängt, auf mindestens einem Steuerparameter (Cj) basiert und aus einer Vielzahl von verschiedenen Funktionsstücken besteht, wobei jedes Funktionsstück der Vielzahl verschiedener Funktionsstücke auf einen jeweiligen Bereich der mindestens einen Signalcharakteristik (Fi) bezogen ist, wobei das Verarbeitungsmodul (18) dazu eingerichtet ist, die von dem Erfassungsmodul (20) aufgezeichneten Aufzeichnungen von neuronalen Aktivitätssignalen basierend auf einer zweiten Steuerlogik (23) zu verarbeiten und den mindestens einen Steuerparameter (Cj) der ersten Steuerlogik (22) basierend auf mindestens einer gemäß der zweiten Steuerlogik (23) verarbeiteten Aufzeichnung von neuronalen Aktivitätssignalen einzustellen.

2. Vorrichtung (10) nach Anspruch 1, wobei das Verarbeitungsmodul (18) dazu eingerichtet ist, den mindestens einen Stimulationsparameter (A) eines Stimulationssignals basierend auf einer ersten Zeitreihe von gesammelten Aufzeichnungen von neuronalen Aktivitätssignalen ($F_1$) einzustellen und den mindestens einen Steuerparameter (Cj) der ersten Steuerlogik (22) basierend auf einer zweiten Zeitreihe von gesammelten Aufzeichnungen von neuronalen Aktivitätssignalen ($F_2$) einzustellen, wobei die erste Zeitreihe kürzer als die zweite Zeitreihe ist.

3. Vorrichtung (10) nach Anspruch 2, wobei die zweite Zeitreihe der gesammelten Aufzeichnungen von neuronalen Aktivitätssignalen, auf deren Grundlage mindestens ein Steuerparameter (Cj) der ersten Steuerlogik (22) eingestellt wird, eine spektrale Leistungszeitreihe ist, die über Minuten oder Stunden oder Tage oder Wochen oder Monate gesammelt wird.

4. Vorrichtung (10) nach einem der Ansprüche 1 bis 3, wobei die zweite Steuerlogik (23) ein Bollinger-Band-Rechner ist, wobei ein erster Steuerparameter (Cj) der ersten Steuerlogik (22) gleich einer oberen Bandgrenze und ein zweiter Steuerparameter ($C_2$) der ersten Steuerlogik (22) gleich einer unteren Bandgrenze eingestellt ist; oder

wobei die zweite Steuerlogik (23) ein unüberwachtes Lernmodell ist, welches dazu eingerichtet ist, ein Cluster der Aufzeichnungen von neuronalen Aktivitätssignalen basierend auf mindestens einer Signalcharakteristik (Fi) für jeden Steuerparameter (Cj) der ersten Steuerlogik (22) zu bilden, wobei jeder Steuerparameter (Cj) einem Schwerpunkt eines jeweiligen Clusters entspricht; oder

wobei die zweite Steuerlogik (23) ein zeitbasierter Fuzzy-Controller ist, welcher dazu eingerichtet ist, mindestens einen Steuerparameter (Cj) der ersten Steuerlogik (22) basierend auf der mindestens einen Signalcharakteristik der Aufzeichnungen von neuronalen Aktivitätssignalen und einem zugehörigen Zeitfenster des Tages, während welchem die Aufzeichnungen von neuronalen Aktivitätssignalen erfasst wurden, zu schätzen.

5. Vorrichtung (10) nach einem der vorhergehenden Ansprüche, wobei mindestens ein Funktionsstück der Vielzahl von Funktionsstücken der ersten Steuerlogik (22) eine Funktion in Abhängigkeit von der mindestens einen Signalcharakteristik (Fi) der Aufzeichnungen von neuronalen Aktivitätssignalen ist, vorzugsweise eine lineare Funktion der mindestens einen Signalcharakteristik (Fi) der Aufzeichnungen von neuronalen Aktivitätssignalen.

6. Vorrichtung (10) nach einem der vorhergehenden Ansprüche, wobei die erste Steuerlogik umfasst:

- ein erstes Funktionsstück, in dem der mindestens eine Stimulationsparameter (A) proportional zu der mindestens einen Signalcharakteristik (Fi) ist, wobei das erste Funktionsstück auf einen ersten Bereich der mindestens einen Signalcharakteristik bezogen ist ($C_2 < F_i < C_1$);

- ein zweites Funktionsstück, welches eine obere Grenze (Amax) des mindestens einen Stimulationsparameters (A) definiert, wobei das zweite Funktionsstück auf einen zweiten Bereich der mindestens einen Signalcharakte-

ristik ($F_i > C_1$) bezogen ist; und
- ein drittes Funktionsstück, welches eine Untergrenze (Amin) des mindestens einen Stimulationsparameters (A) definiert, wobei das dritte Funktionsstück auf einen dritten Bereich der mindestens einen Signalcharakteristik ($F_i < C_2$) bezogen ist.

7. Vorrichtung (10) nach Anspruch 6, wobei die erste Steuerlogik (22)

$$A(F) = \begin{cases} A_{max} & for\ F_1 > C_1(F_2) \\ \dfrac{F_1 - C_2}{C_1 - C_2} * (A_{max} - A_{min}) + A_{min} & for\ C_2 < F_1 < C_1 \\ A_{min} & for\ F_1 < C_2(F_2) \end{cases}$$

ist
wobei der erste Bereich einer ersten Zeitreihe von gesammelten neuronalen Aktivitätssignalcharakteristiken ($F_1$) der Signalcharakteristik eine obere ($C_1$) und eine untere ($C_2$) Bereichsgrenze umfasst, die Steuerparameter der ersten Steuerlogik (22) sind, die auf der Grundlage einer zweiten Zeitreihe von gesammelten neuronalen Aktivitätssignalcharakteristiken ($F_2$) gemäß der zweiten Steuerlogik (23) eingestellt werden.

8. Vorrichtung (10) nach einem der vorhergehenden Ansprüche, wobei die Verarbeitungs- und Stimulationseinheit (14) dazu eingerichtet ist, spektrale Charakteristika innerhalb eines Frequenzbandes der Aufzeichnungen von neuronalen Aktivitätssignalen zu extrahieren, die während eines vordefinierten Zeitraums aufgezeichnet sind, wobei das Frequenzband vorzugsweise ein Niederfrequenzband, das Alpha-Frequenzband, das Beta-Frequenzband oder Gamma-Frequenzen ist; und
wobei das mindestens eine Signalcharakteristikum der Aufzeichnungen von neuronalen Aktivitätssignalen ein spektrales Charakteristikum der Aufzeichnungen von neuronalen Aktivitätssignalen innerhalb des Frequenzbandes ist, vorzugsweise eine Spektralleistung der Aufzeichnungen von neuronalen Aktivitätssignalen innerhalb des Frequenzbandes.

9. Vorrichtung (10) nach Anspruch 8, wobei die erste Steuerlogik (22) ein erstes Funktionsstück umfasst, in dem der mindestens eine Stimulationsparameter (A) proportional zur Spektralleistung (P) der Aufzeichnungen von neuronalen Aktivitätssignalen innerhalb des Frequenzbandes ist, wobei das erste Funktionsstück auf einen ersten Leistungsbereich $Pmin < P < Pmax$) bezogen ist, der zwischen einer minimalen Spektralleistung (Pmin) und einer maximalen Spektralleistung (Pmax) liegt.

10. Vorrichtung (10) nach Anspruch 9, wobei die erste Steuerlogik (22) ein zweites Funktionsstück umfasst, das eine obere Grenze (Amax) des mindestens einen Stimulationsparameters (A) definiert, wobei sich das zweite Funktionsstück auf einen zweiten Leistungsbereich ($P > Pmax$) bezieht, der an den ersten Leistungsbereich *(Pmin < P < Pmax)* angrenzt; und ein drittes Funktionsstück, das eine untere Grenze (Amin) des mindestens einen Stimulationsparameters (A) definiert, wobei sich das dritte Funktionsstück auf einen dritten Leistungsbereich ($P < Pmin)$ bezieht, der an den ersten Leistungsbereich *(Pmin < P < Pmax)* angrenzt.

11. Vorrichtung (10) nach Ansprüche 9 oder 10, wobei die erste Steuerlogik (22)

$$A(P) = \begin{cases} A_{max} & for\ P > Pmax(P_j) \\ \dfrac{P - Pmin(P_j)}{Pmax(P_j) - Pmin(P_j)} * (A_{max} - A_{min}) + A_{min} & for\ Pmin(P_j) < P < Pmax(P_j) \\ A_{min} & for\ P < Pmin(P_j) \end{cases}$$

ist
wobei Amin und Amax jeweils eine untere und eine obere Grenze des Stimulationsparameters (A) sind, und
wobei die minimale Spektralleistung (Pmin) und die maximale Spektralleistung (Pmax) Steuerparameter der ersten Steuerlogik (22) sind, welche basierend auf mindestens einer Aufzeichnung von neuronalen Aktivitätssignalen eingestellt sind, welche gemäß der zweiten Steuerlogik (23) verarbeitet werden.

12. Vorrichtung (10) nach einem der Ansprüche 8 bis 11 in Abhängigkeit von Anspruch 4, wobei die obere Bandgrenze und die untere Bandgrenze eine Zahl (k) einer mittleren Standardabweichung ($\overline{\sigma}$) positiv und negativ von einer Durchschnittsleistung ($\overline{P}$), entfernt sind, die beide mit gleitenden Zeitperioden berechnet werden, wobei vorzugsweise die Durchschnittsleistung ($\overline{P}$) als einfacher gleitender Durchschnittswert der Spektralleistung der Aufzeichnungen von neuronalen Aktivitätssignalen innerhalb des Frequenzbandes oder als exponentieller gleitender Durchschnittswert der Spektralleistung der Aufzeichnungen der neuronalen Aktivitätssignale innerhalb des Frequenzbandes berechnet wird.

**13.** Vorrichtung (10) nach einem der Ansprüche 4 bis 10, wobei der mindestens eine Steuerparameter (Cj) der ersten Steuerlogik (22) gleich dem Durchschnittswert von Signalcharakteristika gesetzt wird, die von der zweiten Steuerlogik (23) basierend auf dem Zeitfenster des Tages, während dem die Aufzeichnungen von neuronalen Aktivitätssignalen erfasst wurden, gruppiert sind.

**14.** Vorrichtung (10) nach einem der vorhergehenden Ansprüche, wobei das Verarbeitungsmodul (18) ferner dazu eingerichtet ist, den mindestens einen Steuerparameter (Cj) basierend auf mindestens einer Aufzeichnung von neuronalen Aktivitätssignalen zu initialisieren, die von dem Erfassungsmodul (20) erfasst wird.

**15.** Vorrichtung (10) nach einem der vorhergehenden Ansprüche, wobei die Verarbeitungs- und Stimulationseinheit (14) dazu eingerichtet ist, ein Stimulationsparameterfenster zu definieren, das zwischen einer unteren Grenze (Amin) des mindestens einen Stimulationsparameters (A) und einer oberen Grenze (Amax) des mindestens einen Stimulationsparameters (A) liegt.

**Revendications**

**1.** - Dispositif (10) destiné au traitement adaptatif de maladies neurologiques comprenant :

une électrode implantable (11) configurée pour détecter des signaux d'activité neuronale et appliquer des signaux de stimulation électrique, et
une unité de traitement et de stimulation (14) connectée à l'électrode implantable (11), l'unité de traitement et de stimulation comprenant au moins :

un module de stimulation (16) configuré pour générer un signal de stimulation à transporter sur l'électrode implantable (11), le signal de stimulation étant **caractérisé par** au moins un paramètre de stimulation ;
un module d'acquisition (20) configuré pour enregistrer des enregistrements de signaux d'activité neuronale des signaux d'activité neuronale détectés par l'électrode implantable ; et
un module de traitement (18) configuré pour traiter les enregistrements de signaux d'activité neuronale enregistrés par le module d'acquisition (20) sur la base d'une première logique de commande (22) et pour accorder l'au moins un paramètre de stimulation (A) sur la base d'au moins un enregistrement de signaux d'activité neuronale traité selon la première logique de commande (22), la première logique de commande étant une fonction dépendant d'au moins une caractéristique de signal (Fi) des enregistrements de signaux d'activité neuronale, étant basée sur au moins un paramètre de commande (Cj) et étant constituée d'une pluralité de différents éléments de fonction, chaque élément de fonction de la pluralité de différents éléments de fonction étant corrélé à une plage respective de l'au-moins une caractéristique de signal (Fi), le module de traitement (18) étant configuré pour traiter les enregistrements de signaux d'activité neuronale enregistrés par le module d'acquisition (20) sur la base d'une seconde logique de commande (23) et pour accorder l'au moins un paramètre de commande (Cj) de la première logique de commande (22) sur la base d'au moins un enregistrement de signaux d'activité neuronale traité conformément à la seconde logique de commande (23).

**2.** - Dispositif (10) selon la revendication 1, dans lequel le module de traitement (18) est configuré pour accorder l'au moins un paramètre de stimulation (A) d'un signal de stimulation sur la base d'une première série temporelle d'enregistrements de signaux d'activité neuronale collectés ($F_1$) et pour accorder l'au moins un paramètre de commande (Cj) de la première logique de commande (22) sur la base d'une seconde série temporelle d'enregistrements de signaux d'activité neuronale collectés ($F_2$), la première série temporelle étant plus courte que la seconde série temporelle.

**3.** - Dispositif (10) selon la revendication 2, dans lequel la seconde série temporelle d'enregistrements de signaux d'activité neuronale collectés sur la base de laquelle au moins un paramètre de commande (Cj) de la première logique de commande (22) est accordé est une série temporelle de puissance spectrale collectée sur des minutes ou des heures ou des jours ou des semaines ou des mois.

4. - Dispositif (10) selon l'une quelconque des revendications 1 à 3, dans lequel la seconde logique de commande (23) est un calculateur de bandes de Bollinger, avec un premier paramètre de commande ($C_1$) de la première logique de commande (22) qui est réglé égal à une limite de bande supérieure et un second paramètre de commande ($C_2$) de la première logique de commande (22) qui est réglé égal à une limite de bande inférieure ; ou

dans lequel la seconde logique de commande (23) est un modèle d'apprentissage non supervisé configuré pour établir une grappe des enregistrements de signaux d'activité neuronale sur la base de son au moins une caractéristique de signal (Fi) pour chaque paramètre de commande (Cj) de la première logique de commande (22), chaque paramètre de commande (Cj) correspondant à un centroïde d'une grappe respective ; ou
dans lequel la seconde logique de commande (23) est un contrôleur flou basé sur le temps configuré pour estimer au moins un paramètre de commande (Cj) de la première logique de commande (22) sur la base de l'au moins une caractéristique de signal des enregistrements de signaux d'activité neuronale et d'un créneau horaire correspondant de la journée au cours duquel les enregistrements de signaux d'activité neuronale ont été acquis.

5. - Dispositif (10) selon l'une quelconque des revendications précédentes, dans lequel au moins un élément de fonction de la pluralité d'éléments de fonction de la première logique de commande (22) est une fonction dépendant de l'au moins une caractéristique de signal (Fi) des enregistrements de signaux d'activité neuronale, de préférence une fonction linéaire de l'au moins une caractéristique de signal (Fi) des enregistrements de signaux d'activité neuronale.

6. - Dispositif (10) selon l'une quelconque des revendications précédentes, dans lequel la première logique de commande comprend :

- un premier élément de fonction dans lequel l'au moins un paramètre de stimulation (A) est proportionnel à l'au moins une caractéristique du signal (Fi), le premier élément de fonction étant corrélé à une première plage de l'au moins une caractéristique de signal ($C_2 < F_i < C_1$) ;
- un deuxième élément de fonction définissant une limite supérieure (Amax) de l'au moins un paramètre de stimulation (A), le deuxième élément de fonction étant corrélé à une deuxième plage de l'au moins une caractéristique de signal ($F_i > C_1$) ; et
- un troisième élément de fonction définissant une limite inférieure (Amin) de l'au moins un paramètre de stimulation (A), le troisième élément de fonction étant corrélé à une troisième plage de l'au moins une caractéristique de signal ($F_i < C_2$).

7. - Dispositif (10) selon la revendication 6, dans lequel la première logique de commande (22) est

$$A\left(F\right) = \begin{cases} A_{max} & pour\ F_1 > C_1(F_2) \\ \dfrac{F_1 - C_2}{C_1 - C_2} * (A_{max} - A_{min}) + A_{min} & pour\ C_2 < F_1 < C_1 \\ A_{min} & pour\ F_1 < C_2(F_2) \end{cases}$$

dans laquelle la première plage d'une première série temporelle de caractéristiques de signaux d'activité neuronale collectés ($F_1$) de la caractéristique de signal comprend une limite de plage supérieure ($C_1$) et une limite de plage inférieure ($C_2$) qui sont des paramètres de commande de la première logique de commande (22) accordés sur la base d'une seconde série temporelle de caractéristiques de signaux d'activité neuronale collectés ($F_2$) conformément à la seconde logique de commande (23).

8. - Dispositif (10) selon l'une quelconque des revendications précédentes, dans lequel l'unité de traitement et de stimulation (14) est configurée pour extraire des caractéristiques spectrales dans une bande de fréquence des enregistrements de signaux d'activité neuronale qui sont enregistrés pendant une période de temps prédéfinie, la bande de fréquence étant, de préférence, une bande de basse fréquence, la bande de fréquence alpha, la bande de fréquence bêta ou les fréquences gamma ; et
dans lequel l'au moins une caractéristique de signal des enregistrements de signaux d'activité neuronale est une caractéristique spectrale des enregistrements de signaux d'activité neuronale dans la bande de fréquence, de préférence une puissance spectrale des enregistrements de signaux d'activité neuronale dans la bande de fréquence.

9. - Dispositif (10) selon la revendication 8, dans lequel la première logique de commande (22) comprend un premier élément de fonction dans lequel l'au moins un paramètre de stimulation (A) est proportionnel à la puissance spectrale (P) des enregistrements de signaux d'activité neuronale dans la bande de fréquence, le premier élément de fonction étant corrélé à une première plage de puissance (*Pmin<P <Pmax*) comprise entre une puissance spectrale minimale (*Pmin*) et une puissance spectrale maximale (*Pmax*).

10. - Dispositif (10) selon la revendication 9, dans lequel la première logique de commande (22) comprend un deuxième élément de fonction définissant une limite supérieure (Amax) de l'au moins un paramètre de stimulation (A), le deuxième élément de fonction étant corrélé à une deuxième plage de puissance (*P>Pmax*) contiguë à la première plage de puissance (*Pmin<P<Pmax*) ; et un troisième élément de fonction définissant une limite inférieure (Amin) de l'au moins un paramètre de stimulation (A), le troisième élément de fonction étant corrélé à une troisième plage de puissance (*P<Pmin*) contiguë à la première plage de puissance (*Pmin<P<Pmax*).

11. - Dispositif (10) selon la revendication 9 ou 10, dans lequel la première logique de commande (22) est

$$A\left(P\right) = \begin{cases} A_{max} & pour\ P > Pmax(P_j) \\ \dfrac{P - Pmin(P_j)}{Pmax(P_j) - Pmin\ (P_j)} * (A_{max} - A_{min}) + A_{min} & pour\ Pmin(P_j) < P < Pmax\ (P_j) \\ A_{min} & pour\ P < Pmin(P_j) \end{cases}$$

Amin et Amax étant respectivement une limite inférieure et une limite supérieure du paramètre de stimulation (A), et

dans laquelle la puissance spectrale minimale (Pmin) et la puissance spectrale maximale (Pmax) sont des paramètres de commande de la première logique de commande (22) accordés sur la base d'au moins un enregistrement de signaux d'activité neuronale traité conformément à la seconde logique de commande (23).

12. - Dispositif (10) selon l'une quelconque des revendications 8 à 11 en dépendance de la revendication 4, dans lequel la limite de bande supérieure et la limite de bande inférieure sont un nombre (k) d'un écart type moyen ($\overline{\sigma}$) positivement et négativement éloigné d'une puissance moyenne ($\overline{P}$), les deux étant calculés avec des périodes de temps glissantes, la puissance moyenne ($\overline{P}$) étant, de préférence, calculée en tant que moyenne mobile simple de la puissance spectrale des enregistrements de signaux d'activité neuronale dans la bande de fréquence ou en tant que moyenne mobile exponentielle de la puissance spectrale des enregistrements de signaux d'activité neuronale dans la bande de fréquence.

13. - Dispositif (10) selon l'une quelconque des revendications 4 à 10, dans lequel l'au moins un paramètre de commande (Cj) de la première logique de commande (22) est réglé égal à la valeur moyenne de caractéristiques de signal regroupées par la seconde logique de commande (23) sur la base du créneau horaire de la journée pendant lequel les enregistrements de signaux d'activité neuronale ont été acquis.

14. - Dispositif (10) selon l'une quelconque des revendications précédentes, dans lequel le module de traitement (18) est en outre configuré pour initialiser l'au moins un paramètre de commande (Cj) sur la base d'au moins un enregistrement de signaux d'activité neuronale acquis par le module d'acquisition (20).

15. - Dispositif (10) selon l'une quelconque des revendications précédentes, dans lequel l'unité de traitement et de stimulation (14) est configurée pour définir une fenêtre de paramètres de stimulation comprise entre une limite inférieure (Amin) de l'au moins un paramètre de stimulation (A) et une limite supérieure (Amax) de l'au moins un paramètre de stimulation (A).

FIG. 1

FIG. 2

21

Processing and
extracting band
power (P)

P

22

Piecewise
function

$P_{max}$     $P_{min}$

P

Bollinger Bands

23

FIG. 3A

21

Processing and
extracting band
power (P)

P

22

Piecewise
function

$P_{max}$     $P_{min}$

P

Unsupervised
learning model

23

FIG. 3B

21

Processing end
extract band
power (P)

P

22

Piecewise
function

$P_{max}$    $P_{min}$

P

Time-Based
Fuzzy controller

23

FIG. 3C

100

101

Read neural signals

Process and extract
neural signal features

102

Adjust stimulation
parameters based on 1st
and 2nd control policy

103

FIG. 4A

200

101

Read neural signals

Process and extract
neural signal
features

102

1st
control
policy
initialized
?

no

yes

201

Adjust stimulation
parameters based on 1st
and 2nd control logic

Process and extract
neural signal features to
initialize 1st control
logic

103

Calculate first set of
stimulation parameters

202

Start Stimulation

203

FIG. 4B

300

Determine the
frequency band around
an oscillatory peak — 301

↓

Determine the
oscillatory power in the
frequency band — 302

↓

Determine the
background power in
the frequency band — 303

↓

Set Pmax equal to the
oscillatory band power — 304

↓

Set Pmin equal to the
background band
power — 305

FIG. 5

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018078770 A
- US 2020108253 A
- US 2012016435 A